# DEMANDE DE BREVET EUROPEEN

(11) **EP 3 536 229 A1**
(43) Date de publication de la demande: **11.09.2019**
(21) Numéro de dépôt: 19305237.0
(22) Date de dépôt: 28.02.2019
(51) Int. Cl.: A61B 5/00

(54) **DISPOSITIF DE QUANTIFICATION DU TAUX DE LIPIDES ET/OU DE PROTEINES DANS LE TISSU HEPATIQUE**

(30) Priorité: 09.03.2018 FR 1852044
(71) Demandeur: Institut Georges Lopez, 69380 Lissieu (FR)
(72) Inventeur: CASTRO-BENITEZ, Carlos, 75005 Paris (FR)
(74) Mandataire: Cabinet Laurent & Charras

(57) **Abrégé**

L'invention concerne un dispositif de quantification (10) du taux de lipides et/ou de protéines présents dans des tissus hépatiques (11) comportant :
- une source de lumière (12), comportant au moins une lampe à vide au tungstène d'une puissance totale comprise entre 0.5 et 2 watts, d'une luminosité totale comprise entre 1000 et 2000 lumens et d'une température de couleur totale comprise entre 6000 et 10000 kelvins ;
- un capteur photosensible (13), comportant une longueur d'onde de sensibilité comprise entre 800 nm et 2450 nm, configuré pour capter ladite lumière (Ri) émise par ladite source de lumière après diffraction dans des tissus hépatiques ;
- des moyens d'extraction d'un spectre de diffraction de ladite lumière en fonction d'une image captée par ledit capteur photosensible ; et
- des moyens d'analyse dudit spectre pour déterminer un taux de lipides et/ou de protéines.

## Description

### DOMAINE TECHNIQUE

L'invention concerne un dispositif de quantification du taux de lipides et/ou de protéines dans le tissu hépatique.

Plus particulièrement, l'invention concerne un dispositif permettant d'estimer le taux de stéatose dans le foie. L'invention trouve une application particulièrement avantageuse pour améliorer les conditions de transplantation du foie.

### ART ANTERIEUR

La première étape dans le cadre de la transplantation du foie consiste à rechercher si le greffon, c'est-à-dire l'organe et ses structures anatomiques adjacentes, est sain. Le premier examen est généralement réalisé en amont de la transplantation en étudiant le dossier médical du donneur. Cependant, le dossier médical est souvent insuffisant pour permettre au chirurgien d'estimer la qualité du greffon.

Au moment du prélèvement du foie par extraction depuis le donneur, le chirurgien effectue un second examen visuel par palpation et évaluation de la consistance, de la couleur et de la taille du greffon. En France, environ 100 greffons par an ne sont pas prélevés car le chirurgien juge que le foie n'est pas conforme en raison, notamment, de son aspect stéatosique.

Une fois le greffon prélevé, il est transporté vers le centre où le receveur est en attente d'une greffe. Ensuite, le greffon est une nouvelle fois examiné de visu par le chirurgien qui réalise la greffe. Celui-ci palpe et regarde la couleur et la consistance du greffon. En France, ce dernier examen conduit au rejet d'environ 30 greffons par an car le chirurgien en charge de la transplantation juge que l'aspect du foie n'est pas conforme, notamment en raison d'une stéatose.

La stéatose hépatique est une pathologie du foie associée à une surcharge de lipides, essentiellement les triglycérides, dans le cytoplasme des hépatocytes. La prévalence de cette pathologie est de 20% à 30% dans les pays développés. Il s'agit donc d'une pathologie fréquente, dont la prévalence tend à augmenter avec l'accroissement des cas d'obésité et de diabète.

Deux types de stéatose hépatique sont observés, la stéatose micro-vacuolaire à l'étiologie variée et se caractérise par la présence de plusieurs petites vacuoles lipidiques dans le cytoplasme et dont la présence n'induit pas de déplacement du noyau des hépatocytes. Le second type de stéatose, le plus sévère, est la stéatose macro-vacuolaire dont l'une des étiologies est une consommation excessive d'alcool. Ce type de stéatose se caractérise par une grande vacuole lipidique unique qui déplace le noyau des hépatocytes.

Bien que les chirurgiens soient rompus à ce genre d'observations, cet examen visuel et de palpation est souvent difficile à réaliser en raison, notamment, de la présence de sang autour du foie lors du prélèvement. De plus, cet examen est subjectif, expliquant que les résultats peuvent diverger d'un chirurgien à un autre.

La stéatose se caractérise éventuellement par la présence d'une accumulation de protéines dans le cytoplasme des hépatocytes. A titre d'exemple, les cellules du foie peuvent contenir en excès des enzymes telles que les transaminases (alanine-aminotransférase/ALAT ou aspartate-aminotransférase/ASAT) ou les gamma-glutamyl-transpeptidases (Gamma-GT). Dans un contexte de stéatose, les hépatocytes peuvent également se caractériser par la présence de corps de Mallory, c'est-à-dire, des agrégats intrahépatocytaires éosinophiles de microfilaments intermédiaires de prékératine provenant du cytosquelette de l'hépatocyte.

Ainsi, une méthode plus efficace pour estimer le taux de stéatose d'un greffon a été mise à l'étude.

Des études récentes ont montré qu'il est possible de quantifier la présence de stéatose dans un greffon par diffraction d'une lumière infrarouge dans le greffon : « Chemical Imaging on Liver Steatosis Using Synchrotron Infrared and ToF-SIMS Microspectroscopies », Le Naour &all, PLoS ONE, october 2009, volume 4, issue 10, e7408, « In Situ Chemical Composition Analysis of Cirrholis by Combining Synchrotron Fourier Transform Infrared and Synchrotron X-ray Fluorescence Microscopies on the Same Tissue Section », Le Naour & all, analytical chemistry, 2012, 84, 10260-10266, et, « Discrimination of cirrhotic nodules, dysplastic lesions and hepatocellular carcinoma by their vibrational signature », Peng & all, J. Transi. Med., 2016, 14:19. Les résultats de ces travaux sont également repris dans le document US 2008/306337.

Pour mener ces études, une biopsie du greffon est réalisée au moment du prélèvement du greffon. L'échantillon ainsi recueilli est transmis à un laboratoire de recherche, qui analyse la diffraction de la lumière infrarouge dans la biopsie.

Cette analyse est particulièrement complexe. En effet, pour obtenir la lumière infrarouge recherchée, ces études décrivent l'utilisation du synchrotron « SOLEIL » permettant de fournir une source lumineuse d'une haute brillance dans la région spectrale 1-100 microns avec une optimisation de la détection dans la région 2.5-100 microns. Cette source spécifique est couplée à un microscope infrarouge FTIR.

Ces études en laboratoire permettent de détecter précisément le taux de stéatose d'un échantillon et, ainsi, de déterminer si l'échantillon, et par généralisation le foie entier, est sain.

Avantageusement, si le foie contient moins de 5% de lipides, cet organe est considéré sain.

Cependant, le taux de stéatose peut varier d'une région à une autre du même foie. Ainsi, les mesures réalisées sur une biopsie du foie ne peuvent pas se généraliser à l'organe entier et ne permettent pas de quantifier son état de santé.

En outre, l'utilisation d'un microscope à infrarouge FTIR pour réaliser ces mesures impose l'utilisation d'un laboratoire spécifiquement équipé, souvent distant des zones de prélèvement et de transplantation du greffon.

Enfin, compte tenu de la fragilité du greffon, il est nécessaire de limiter au maximum le délai entre le prélèvement et la transplantation du greffon. Dans ces conditions, la durée du transport d'un échantillon de greffon jusqu'au laboratoire d'analyse spécifiquement équipé peut limiter les possibilités de transplantation du greffon.

Optimiser les conditions d'analyse du tissu hépatique permettrait de raccourcir ce délai.

Le document US 2014/131578 décrit un spectromètre portable intégrant une lampe à vide au tungstène. Ce spectromètre est plus particulièrement décrit pour être utilisé dans l'industrie pharmaceutique, en particulier pour analyser les matières premières.

Au vue de la spécificité des sources lumineuses utilisables sur des greffons telles que décrites dans les études scientifiques précédemment mentionnées, le spectromètre illustré dans le document US 2014/131578 semble contre-indiqué pour quantifier le taux de lipides et/ou de protéines présents dans des tissus hépatiques

Le problème technique de l'invention consiste donc à améliorer le processus existant d'analyse d'un tissu hépatique par diffraction.

### EXPOSE DE L'INVENTION

Le Demandeur a découvert que la diffraction d'une lumière large spectre permet d'obtenir des diffractions révélatrices du taux de lipides et/ou de protéines présents dans les tissus hépatiques. Ce faisant, l'invention permet de s'affranchir de l'utilisation d'une lumière infrarouge au spectre très spécifique et permet de réaliser un dispositif d'analyse beaucoup plus simple et de taille beaucoup plus réduite. Il s'ensuit que le dispositif d'analyse peut être disposé dans le bloc opératoire de sorte qu'un examen du greffon peut être réalisé par diffraction de lumière à large spectre dans le greffon avant même l'extraction du greffon, et sans effectuer une biopsie de l'organe. De plus, plusieurs analyses peuvent être réalisées sur des multiples régions du greffon sans occasionner de lésions du greffon, c'est-à-dire sans réaliser de biopsie.

A cet effet, selon un premier aspect, l'invention concerne un dispositif de quantification du taux de lipides et/ou de protéines présents dans des tissus hépatiques, ledit dispositif comportant :
- une source de lumière configurée pour projeter ladite lumière sur des tissus hépatiques à analyser ;
- un capteur photosensible configuré pour capter ladite lumière émise par ladite source de lumière après diffraction dans lesdits tissus hépatiques ;
- des moyens d'extraction d'un spectre de diffraction de ladite lumière en fonction d'une image captée par ledit capteur photosensible ; et
- des moyens d'analyse dudit spectre pour déterminer un taux de lipides et/ou de protéines présents dans lesdits tissus hépatiques.

L'invention se caractérise en ce que :
- ladite source de lumière se présente sous la forme d'au moins une lampe à vide au tungstène d'une puissance totale comprise entre 0,5 et 2 watts, d'une luminosité totale comprise entre 1000 et 2000 lumens et d'une température de couleur totale comprise entre 6000 et 10000 kelvins ; et
- ledit capteur photosensible présente une longueur d'onde de sensibilité comprise entre 800 nm et 2450 nm.

Selon un mode de réalisation de l'invention, ledit capteur photosensible présente une longueur d'onde de sensibilité comprise entre 1100 nm et 2450 nm.

Contre toute attente, l'utilisation d'une lampe à vide au tungstène avec une puissance adaptée est suffisante pour remplacer la source infrarouge d'un synchrotron car les diffractions révélées par le capteur photosensible permettent d'estimer le taux de lipides et/ou de protéines présents dans des tissus hépatiques.

La taille d'une lampe à vide au tungstène étant très inférieure à la taille des sources lumineuses de l'état de la technique, au moins avec un rapport de 100, il est possible de réaliser une implémentation totalement différente des solutions de l'état de la technique. En particulier, il n'est plus nécessaire d'utiliser un laboratoire avec des équipements spécifiques dédiés pour réaliser l'analyse. Par exemple, la source de lumière et le capteur peuvent être implémentés sur un dispositif électronique de la taille d'un téléphone intelligent.

Selon un mode de réalisation, le dispositif comporte deux lampes à vide au tungstène. Ce mode de réalisation permet de faciliter l'implémentation de la source de lumière de sorte à obtenir les caractéristiques totales recherchées pour la source de lumière.

Selon un mode de réalisation, ladite source de lumière et ledit capteur photosensible sont disposés à une distance maximum de 3 cm l'un de l'autre. Ainsi, la source de lumière est très proche du capteur photosensible et la lumière est captée par réflexion de la lumière sur les tissus hépatiques.

Cette solution est particulièrement avantageuse par rapport à l'état de la technique qui utilise une source de lumière disposée au-dessus d'un échantillon et un capteur disposé au-dessous de l'échantillon. En effet, cette solution permet d'utiliser un seul boîtier électronique pour réaliser l'émission et la réception de la lumière.

Selon un mode de réalisation, ladite source de lumière est alimentée par une tension comprise entre 3 et 15 Volts. Ce mode de réalisation permet d'alimenter la source de lumière avec une faible quantité d'énergie et, ainsi, de faciliter l'intégration de l'alimentation de la source de lumière.

Selon un mode de réalisation, ladite source de lumière est alimentée par un courant compris entre 0,1 et 0,4 Ampères. Ce mode de réalisation permet d'alimenter la source de lumière avec une faible quantité d'énergie et, ainsi, de faciliter l'intégration de l'alimentation de la source de lumière.

Selon un mode de réalisation, ledit capteur photosensible correspond à un détecteur de type InGas. Ce mode de réalisation permet de détecter efficacement la lumière diffractée tout en permettant l'intégration du capteur photosensible dans un dispositif portable.

Selon un second aspect, l'invention concerne un procédé de quantification du taux de lipides et/ou de protéines présents dans des tissus hépatiques d'un donneur au moyen d'un dispositif d'analyse selon le premier aspect de l'invention, ledit procédé comportant les étapes suivantes :
- positionnement de ladite source de lumière proche des tissus hépatiques à analyser ;
- émission d'une lumière sur des tissus hépatiques à analyser ;
- réception de la lumière émise par ladite source de lumière et diffractée dans lesdits tissus hépatiques par le capteur photosensible ;
- extraction d'un spectre de diffraction de ladite lumière en fonction d'une image captée par ledit capteur photosensible ; et
- analyse dudit spectre pour déterminer un taux de lipides et/ou de protéines présents dans lesdits tissus hépatiques.

Selon un mode de réalisation, l'étape de positionnement est réalisée avant extraction du donneur, c'est-à-dire lorsque le foie à transplanter est présent dans le corps du donneur.

Selon un mode de réalisation, l'étape d'analyse est réalisée avec des images de référence des tissus hépatiques pour des taux de lipides et/ou de protéines distincts.

Selon un mode de réalisation, ledit procédé comporte une étape d'aide à la décision du prélèvement ou non du foie en fonction du taux de lipides et/ou de protéines présents dans lesdits tissus hépatiques analysés.

### DESCRIPTION SOMMAIRE DES FIGURES

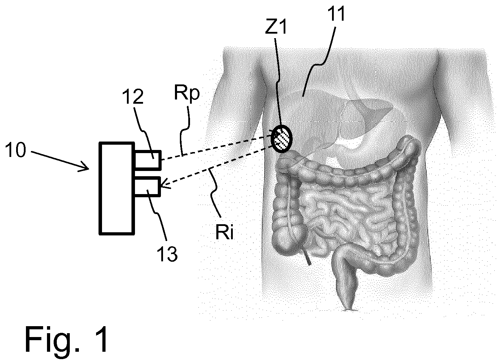

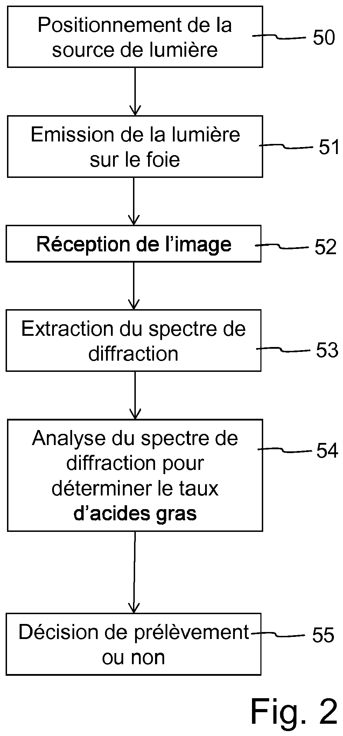

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 et 2 représentent :
- figure 1 : une représentation schématique d'un dispositif de quantification du taux de lipides et/ou de protéines lors d'une utilisation dans un bloc opératoire selon un mode de réalisation de l'invention ; et
- figure 2 : un organigramme d'un procédé de quantification du taux de lipides et/ou de protéines au moyen du dispositif de la figure 1.

### DESCRIPTION DETAILLEE DE L'INVENTION

La figure 1 illustre un donneur disposé sur une table d'opération lors d'une opération de prélèvement du foie. De préférence, le corps du donneur est ouvert pour que le foie soit visible et accessible.

Dans une première étape **50**, illustrée sur la figure 2, un dispositif **10** conforme à l'invention est disposé en regard du foie du donneur, par exemple à une distance comprise entre 10 et 20 cm du foie du donneur. L'utilisateur du dispositif **10**, par exemple un chirurgien ou une infirmière du bloc opératoire (IBODE), peut alors commander le démarrage d'une mesure du taux de lipides et/ou de protéines.

Cette commande peut être réalisée en pressant un bouton poussoir, non représenté, disposé sur le dispositif **10**.

Dans une seconde étape **51**, le dispositif **10** procède à l'émission d'une lumière large spectre **Rp** par l'intermédiaire d'une source de lumière **12** disposée en regard du foie. Cette source de lumière **12** se présente sous la forme d'au moins une lampe à vide au tungstène d'une puissance totale comprise entre 0,5 et 2 watts, d'une luminosité totale comprise entre 1000 et 2000 lumens et d'une température de couleur totale comprise entre 6000 et 10000 kelvins.

De préférence, la source de lumière **12** est réalisée par deux lampes à vide au tungstène à large spectre.

Par exemple, chaque lampe à vide présente une puissance de 0,6 watts pour obtenir la puissance totale dans la plage requise. En variante, le nombre de lampes à vide au tungstène peut être plus important, par exemple trois ou quatre lampes peuvent être utilisées sans changer l'invention. Plus le nombre de lampes est important, plus les lampes sont faciles à réaliser car elles doivent présenter des performances moindres. Cependant, le nombre de lampes augmente l'encombrement du dispositif **10**.

Pour limiter l'encombrement du dispositif **10**, il est préférentiellement recherché des lampes avec des consommations limitées, typiquement comprises entre 3 et 15 Volts et 0,1 et 0,4 Ampères. Ce faisant, il est possible d'utiliser une alimentation avec une taille limitée pour alimenter les lampes. Par exemple, on peut utiliser deux lampes à vide avec une puissance de 0.6 watts et une alimentation de 5 Volts et 0,12 Ampères.

Lorsque la source de lumière **12** émet la lumière à large spectre **Rp**, cette lumière touche une zone **Z1** du foie. Au contact de cette zone **Z1**, la lumière **Rp** se diffracte dans toutes les directions formant ainsi un rayon incident **Ri** en direction du dispositif **10**.

Ce rayon incident **Ri** est capté par un capteur photosensible **13** disposé à côté de la source de lumière **12**, par exemple à une distance maximum de 5 cm.

Dans l'exemple de la figure 1, la source de lumière **12** et le capteur photosensible **13** sont montés sur le même boîtier. En variante, ces éléments optiques peuvent être montés sur deux boîtiers distincts sans changer l'invention.

En outre, ces éléments optiques peuvent également être montés sur un téléphone intelligent configuré pour réaliser les traitements du signal reçu **Ri** ou pour transmettre le signal reçu à un système de traitement.

Le capteur photosensible **13** présente une longueur d'onde de sensibilité comprise entre 800 nm et 2450 nm et, dans un mode de réalisation de l'invention, entre 1100 et 2450 nm. Ainsi, bien que la lumière **Rp** émise par la source de lumière **12** soit large spectre, le capteur photosensible **13** capte au minimum les longueurs d'ondes reçu dans la plage 800 nm et 2450 nm ou entre 1100 et 2450 nm, soit une partie seulement du spectre de la lumière **Rp**. Par exemple, ce capteur photosensible 13 peut correspondre à un détecteur de type InGas.

Dans une étape **52**, ce capteur photosensible **13** acquière une image spectrale des variations de la lumière diffractée **Ri** sur la zone **Z1** du foie. Des traitements d'image sont ensuite mis en oeuvre pour définir la qualité du foie.

Dans une première étape **53** de traitement de l'image du capteur photosensible **13**, le spectre de la lumière diffractée **Ri** est recherché. Ce spectre est comparé, dans une étape **54**, avec des spectres préenregistrés pour différents états du foie. Par exemple, cette comparaison peut être réalisée par un réseau de neurones configuré pour rechercher un maximum de corrélation entre le spectre issu de l'étape **53** et des spectres préenregistrés associés à différents états du foie. De manière classique, ce réseau de neurones devrait subir une étape d'apprentissage dans laquelle des spectres typiques sont enregistrés pour différents états du foie. Par exemple, dix spectres peuvent être enregistrés pour un foie avec un taux de lipides et/ou de protéines de 10%, dix spectres pour un foie avec un taux de lipides et/ou de protéines de 20%, et ainsi de suite jusqu'à 90% de lipides et/ou de protéines.

Ainsi, le réseau de neurones compare le spectre issu de l'étape **53** avec une grande quantité de spectres préenregistrés et recherche le maximum de corrélation.

Cette étape de recherche de corrélation peut, par exemple, être réalisée par un réseau de neurones de type RBF, pour « *Radial Basic Function* » dans la littérature anglo-saxonne. Ce réseau de neurones RBF présente un ensemble de neurones cachés associés à chaque spectre préenregistré. Chaque neurone caché effectue un calcul de corrélation entre le spectre issu de l'étape **53** et un spectre préenregistré.

Chaque neurone caché délivre un score de corrélation entre le spectre issu de l'étape **53** et un spectre préenregistré. Ce score de corrélation indique la ressemblance entre le spectre issu de l'étape **53** et le spectre préenregistré. Un neurone de sortie détermine le maximum des scores de corrélation pour déterminer à quel spectre préenregistré correspond le spectre issu de l'étape **53**. Ce neurone de sortie est préférentiellement associé à un seuil du score de corrélation au-dessous duquel le réseau de neurones considère qu'aucune corrélation acceptable n'est trouvée.

En variante, d'autres architectures et stratégie d'analyse du spectre peuvent être utilisées sans changer l'invention.

Lorsque le spectre est corrélé à un spectre préenregistré, il est recherché le taux de lipides et/ou de protéines associé au spectre préenregistré, par exemple le spectre préenregistré peut être enregistré avec un taux de lipides et/ou de protéines de 20%. Cette mesure du taux de lipides et/ou de protéines peut être réalisée avec les moyens connus d'estimation du taux de lipides et/ou de protéines, par exemple avec les moyens décrits dans les publications : « Chemical Imaging on Liver Steatosis Using Synchrotron Infrared and ToF-SIMS Microspectroscopies », Le Naour &all, PLoS ONE, october 2009, volume 4, issue 10, e7408, « In Situ Chemical Composition Analysis of Cirrholis by Combining Synchrotron Fourier Transform Infrared and Synchrotron X-ray Fluorescence Microscopies on the Same Tissue Section », Le Naour & all, analytical chemistry, 2012, 84, 10260-10266, et, «Discrimination of cirrhotic nodules, dysplastic lesions and hepatocellular carcinoma by their vibrational signature », Peng & all, J. Transi. Med., 2016, 14:19.

Ainsi, le taux de lipides et/ou de protéines du spectre mesuré est déterminé en fonction du taux de lipides et/ou de protéines du spectre le plus proche du spectre mesuré.

Cette estimation du taux de lipides et/ou de protéines est ensuite transmise à l'utilisateur dans une étape **55**. Cette transmission peut être réalisée par un écran LCD disposé sur le dispositif **10** ou directement sur le téléphone de l'utilisateur par une application connectée au dispositif 10.

Pour finir, le chirurgien peut utiliser cette information sur le taux de lipides et/ou de protéines pour estimer avec plus de précision et de rapidité si le foie doit ou non être prélevé du donneur.

Pour conclure, l'invention améliore le processus d'estimation de la qualité du foie d'un donneur. En outre, cette estimation peut être réalisée directement dans le corps du donneur sans réaliser une biopsie et une analyse avec un microscope.

## Revendications

1. Dispositif de quantification (10) du taux de lipides et/ou de protéines présents dans les tissus hépatiques (11), ledit dispositif comportant :
- une source de lumière (12) configurée pour projeter ladite lumière (Rp) sur des tissus hépatiques à analyser (Z1) ;
- un capteur photosensible (13) configuré pour capter ladite lumière (Ri) émise par ladite source de lumière (12) après diffraction dans lesdits tissus hépatiques ;
- des moyens d'extraction d'un spectre de diffraction de ladite lumière en fonction d'une image captée par ledit capteur photosensible (13) ; et
- des moyens d'analyse dudit spectre pour déterminer un taux de lipides et/ou de protéines présents dans lesdits tissus hépatiques ;
***caractérisé en ce que* :**
- ladite source de lumière (12) se présente sous la forme d'au moins une lampe à vide au tungstène d'une puissance totale comprise entre 0.5 et 2 watts, d'une luminosité totale comprise entre 1000 et 2000 lumens et d'une température de couleur totale comprise entre 6000 et 10000 kelvins ; et
- ledit capteur photosensible (13) présente une longueur d'onde de sensibilité comprise entre 800 nm et 2450 nm.

2. Dispositif de quantification selon la revendication 1, ***caractérisé en ce que*** ledit capteur photosensible (13) présente une longueur d'onde de sensibilité comprise entre 1100 nm et 2450 nm.

3. Dispositif de quantification selon la revendication 1 ou 2, ***caractérisé en ce que*** le dispositif comporte deux lampes à vide au tungstène.

4. Dispositif de quantification selon l'une des revendications 1 à 3, ***caractérisé en ce que*** ladite source de lumière (12) et ledit capteur photosensible (13) sont disposés à une distance maximum de 3 cm l'un de l'autre.

5. Dispositif de quantification selon l'une des revendications 1 à 4, ***caractérisé en ce que*** ladite source de lumière (12) est alimentée par une tension comprise entre 3 et 15 Volts.

6. Dispositif de quantification selon l'une des revendications 1 à 5, ***caractérisé en ce que*** ladite source de lumière (12) est alimentée par un courant compris entre 0.1 et 0.4 Ampères.

7. Dispositif de quantification selon l'une des revendications 1 à 6, ***caractérisé en ce que*** ledit capteur photosensible (13) correspond à un détecteur de type InGas.

8. Procédé de quantification du taux d'acides gras et/ou de protéines présents dans des tissus hépatiques (11) d'un donneur au moyen d'un dispositif d'analyse selon l'une des revendications 1 à 7, ***caractérisé en ce que*** ledit procédé comporte les étapes suivantes :
- positionnement (50) de ladite source de lumière (12) proche des tissus hépatiques à analyser (Z1) ;
- émission (51) d'une lumière (Rp) sur des tissus hépatiques à analyser (Z1) ;
- réception (52) de la lumière (Ri) émise par ladite source de lumière (12) et diffractée dans lesdits tissus hépatiques au moyen du capteur photosensible (13) ;
- extraction (53) d'un spectre de diffraction de ladite lumière en fonction d'une image captée par ledit capteur photosensible (13) ; et
- analyse (54) dudit spectre pour déterminer un taux de lipides et/ou de protéines présents dans lesdits tissus hépatiques.

9. Procédé selon la revendication 8, ***caractérisé en ce que*** l'étape de positionnement (50) est réalisée alors que le tissu hépatique est présent dans le corps du donneur, avantageusement avant toute étape de la transplantation.

10. Procédé selon la revendication 8 ou 9, ***caractérisé en ce que*** l'étape d'analyse (54) est réalisée avec des images de référence des tissus hépatiques pour des taux de lipides et/ou de protéines distincts.

11. Procédé selon l'une des revendications 8 à 10, ***caractérisé en ce que*** ledit procédé comporte une étape de décision (55) du prélèvement ou non du foie en fonction du taux de lipides et/ou de protéines présents dans lesdits tissus hépatiques analysés.
